# EUROPEAN PATENT APPLICATION

(11) **EP 4 112 102 A1**
(43) Date of publication of application: **04.01.2023**
(21) Application number: 21775747.5
(22) Date of filing: 08.03.2021
(51) Int. Cl.: A61M 5/28, A61M 39/10

(54) **LOCK ADAPTER, SYRINGE ASSEMBLY, AND PREFILLED SYRINGE**

(30) Priority: 24.03.2020 JP 2020052002
(71) Applicant: TERUMO Kabushiki Kaisha, Tokyo 151-0072 (JP)
(72) Inventor: OGAWA, Junichi, Nakakoma-gun, Yamanashi 409-3853 (JP)
(74) Representative: Casalonga
(86) International application number: PCT/JP2021/008873
(87) International publication number: WO 2021/192945

(57) **Abstract**

Provided are a lock adapter (22), a syringe assembly (50), and a prefilled syringe (10) capable of suppressing variation in position on the tip end side of the lock adapter (22) after being subjected to an autoclave sterilization process. The lock adapter (22) attached to the periphery of a nozzle portion (18) at the tip end of the prefilled syringe (10) and configured slidable in an axial direction of the nozzle portion (18) includes: a cylindrical body portion (23) formed in a cylindrical shape surrounding the periphery of the nozzle portion (18); and a claw portion (30) protruding to an inner peripheral side from protruding from a base end portion (28) of the cylindrical body portion (23) and configured to determine a tip end position of the lock adapter (22) by being caught in an annular rib (32) extending in a circumferential direction of the nozzle portion (18), in which the claw portion (30) is formed in a circumferential shape when viewed from the axial direction and is integrally connected in the circumferential direction.

## Description

### Technical Field

The present invention relates to a lock adapter, a syringe assembly, and a prefilled syringe for an injector.

### Background Art

Prefilled syringes in which a syringe is filled with a medicinal solution in advance have been widely used. In some prefilled syringes, a lock adapter is attached to the periphery of a nozzle portion at the tip end of the syringe, and a cap covering the nozzle portion is screwed into a female screw of the lock adapter for sealing.

JP 2008-35913 A discloses a prefilled syringe having a slidable lock adapter in which the lock adapter is configured slidable in the axial direction. In this prefilled syringe, a slide portion for allowing the lock adapter to slide in the axial direction is provided at a base portion of the nozzle portion. The slide portion is provided with six rotation restricting ribs extending in the axial direction to prevent rotation of the lock adapter, and an annular rib extending in the circumferential direction along a boundary between the slide portion and the base portion of a luer tip.

Furthermore, the lock adapter is provided with six claw portions extending inward. The claw portions of the lock adapter are disposed between the rotation restricting ribs and configured to slide in the axial direction along the outer surface of the slide portion. In addition, when the cap is screwed thereon, the claw portions of the lock adapter are caught by the annular rib, so that the lock adapter is prevented from coming off from the slide portion while withstanding the tightening load of the cap.

### Summary of Invention

In the conventional lock adapter, the claw portions of the lock adapter are fragile, and when the autoclave sterilization process is performed in a state where the cap is screwed thereon, the strength of the claw portions becomes insufficient due to application of heat. As a result, the claw portions might not be able to withstand the load acting from the cap, and thus are deformed.

Variation may thus be increased in the position on the tip end side of the lock adapter after being subjected to the autoclave sterilization process, so that it may be difficult to keep the position within the desired dimensional tolerance.

An object of one embodiment is to provide a lock adapter, a syringe assembly, and a prefilled syringe capable of suppressing variation in position on the tip end side of the lock adapter after being subjected to an autoclave sterilization process.

One aspect of the following disclosure resides in a lock adapter attached to the periphery of a nozzle portion at the tip end of a prefilled syringe and configured slidable in the axial direction of the nozzle portion, the lock adapter including: a cylindrical body portion formed in a cylindrical shape surrounding the periphery of the nozzle portion; and a claw portion protruding to the inner peripheral side from a base end portion of the cylindrical body portion, and configured to determine a tip end position of the lock adapter by being caught in an annular rib extending in the circumferential direction of the nozzle portion, in which the claw portion is formed in a circumferential shape when viewed from the axial direction and is integrally connected in the circumferential direction.

Another aspect resides in a syringe assembly including: a syringe body having a lumen capable of accommodating a medicinal solution; a nozzle portion formed at the tip end of the syringe body; and a lock adapter attached to the outer periphery of the nozzle portion, in which the nozzle portion includes: a slide portion provided on the base end side and on which the lock adapter is slidable in the axial direction; a luer tip provided on the tip end side of the slide portion; and an annular rib extending and protruding in the circumferential direction along a boundary between the luer tip and the slide portion, the lock adapter includes: a cylindrical body portion formed in a cylindrical shape surrounding the periphery of the nozzle portion; and a claw portion protruding to the inner peripheral side from a base end portion of the cylindrical body portion, and configured to determine a tip end position of the lock adapter by being caught in the annular rib extending in the circumferential direction of the nozzle portion, and the claw portion of the lock adapter is formed in a circumferential shape when viewed from the axial direction and is integrally connected in the circumferential direction.

Yet another aspect resides in a prefilled syringe including: a syringe body having a lumen accommodating a medicinal solution; a gasket to be inserted from the base end side of the syringe body to seal the lumen; a nozzle portion formed at the tip end of the syringe body; a lock adapter attached to the outer periphery of the nozzle portion; and a cap to be screwed into the lock adapter to seal the nozzle portion, in which the nozzle portion includes: a slide portion provided on the base end side and on which the lock adapter is slidable in the axial direction; a luer tip provided on the tip end side of the slide portion; and an annular rib extending and protruding in the circumferential direction along a boundary between the luer tip and the slide portion, the lock adapter includes: a cylindrical body portion formed in a cylindrical shape surrounding the periphery of the nozzle portion; and a claw portion protruding to the inner peripheral side from a base end portion of the cylindrical body portion, and configured to determine a tip end position of the lock adapter by being caught in the annular rib extending in the circumferential direction of the nozzle portion, and the claw portion of the lock adapter is formed in a circumferential shape when viewed from the axial direction and is integrally connected in the circumferential direction.

With the lock adapter, the syringe assembly, and the prefilled syringe in the above-mentioned aspects, since the claw portion of the lock adapter is less likely to be deformed during the autoclave sterilization process, it is possible to suppress variation in the position on the tip end side of the lock adapter.

### Brief Description of Drawings

Fig. 1 is a plan view of a prefilled syringe according to one embodiment.
Fig. 2 is a partially enlarged cross-sectional view of the vicinity of a nozzle portion of the prefilled syringe of Fig. 1.
Fig. 3 is a perspective view of the tip end side of a lock adapter of Fig. 2.
Fig. 4 is a perspective view of the base end side of the lock adapter of Fig. 2.
Fig. 5 is a front view of the lock adapter of Fig. 2.
Fig. 6 is a partially enlarged perspective view of the vicinity of the nozzle portion of the prefilled syringe of Fig. 2.
Fig. 7 is an enlarged view of the nozzle portion of the prefilled syringe of Fig. 2 as viewed from the front side.
Fig. 8 is a cross-sectional view taken along line VIII-VIII in Fig. 6.
Fig. 9 is a cross-sectional view of a state in which a cap is screwed into a syringe assembly of Fig. 2.
Fig. 10A is a perspective view of a lock adapter and a nozzle portion of a prefilled syringe according to a comparative example. Fig. 10B is a perspective view of the nozzle portion of Fig. 10A. Fig. 10C is a front view of the lock adapter of Fig. 10A.
Fig. 11 is a front view illustrating claw portions of the lock adapter and a contact portion with an annular rib of the comparative example.
Fig. 12 is a front view illustrating a claw portion of the lock adapter and a contact portion with an annular rib according to one embodiment.
Fig. 13 is a cross-sectional view of the contact portion between the claw portion and the annular rib of the lock adapter according to one embodiment.

### Description of Embodiments

Hereinafter, preferred embodiments of a lock adapter 22, a syringe assembly 50, and a prefilled syringe 10 will be described in detail with reference to the accompanying drawings. It should be noted that in the following description, the side of a nozzle portion 18 of the prefilled syringe 10 is referred to as the tip end or the tip end side, and the side of a flange portion 16 of the prefilled syringe 10 is referred to as the base end or the base end side. In addition, an alternate long and short dash line A in the drawings represents an axis of the prefilled syringe 10, and the direction of the alternate long and short dash line A is referred to as the axial direction.

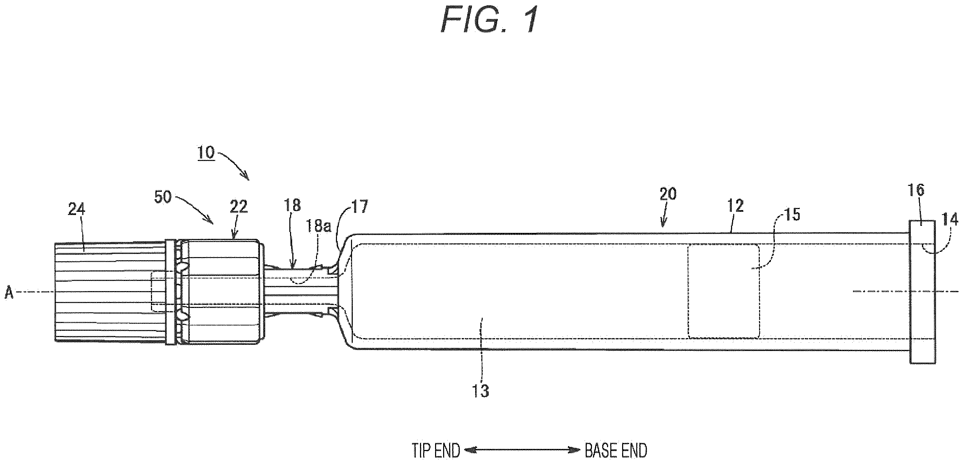

As illustrated in Fig. 1, the prefilled syringe 10 according to the present embodiment includes a syringe body 12 formed in a cylindrical shape, and a lumen 13 extending in the axial direction is formed inside the syringe body 12. A base end opening portion 14 through which the lumen 13 opens is provided on the base end side of the syringe body 12. A gasket 15 is inserted into the lumen 13 from the base end opening portion 14.

The gasket 15 is configured slidable in the axial direction while liquid-tightly sealing the inside of the lumen 13, and a portion of the lumen 13 closer to the tip end side than the gasket 15 is a drug chamber that is liquid-tightly sealed by the gasket 15. The drug chamber of the lumen 13 sealed by the gasket 15 is filled with a medicinal solution or a liquid preparation such as injection water or physiological saline for dilution in advance. The prefilled syringe 10 may be provided to a user in a state where a plunger (not illustrated) is attached, and in such a case, a plunger (pusher) for pressing the gasket 15 may be attached to the gasket 15. As a constituent material of the gasket 15, a rubber material having elasticity, an elastomer material, or the like can be used.

At the base end of the syringe body 12, the flange portion 16 is formed to protrude radially outward. A finger grip (not illustrated) can be attached to the flange portion 16. In the prefilled syringe 10, the finger grip may be attached to the flange portion 16 in advance. In addition, the flange portion 16 and the finger grip may be integrally formed.

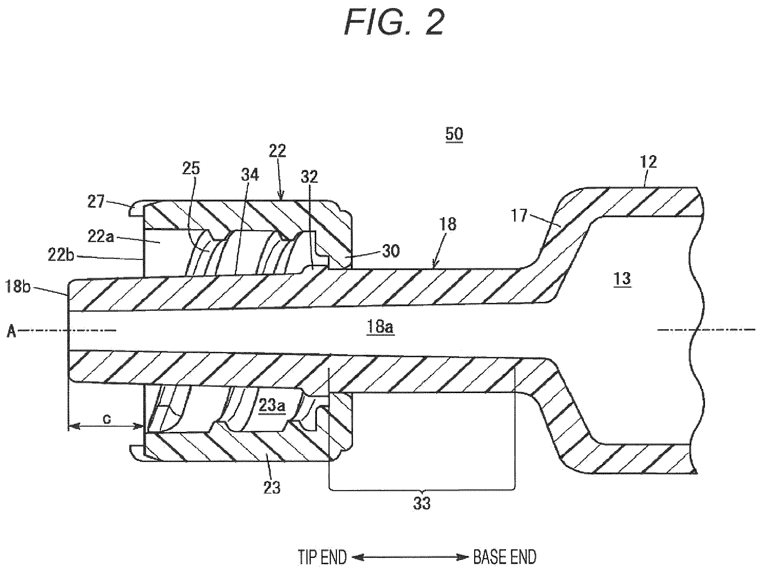

A neck portion 17 having a diameter smaller than that of the syringe body 12 is formed at the tip end of the syringe body 12. From the neck portion 17, the nozzle portion 18 is formed to protrude toward the tip end side. As illustrated in Fig. 2, a flow path 18a is formed inside the nozzle portion 18. The flow path 18a has an opening at a tip end surface 18b of the nozzle portion 18. The base end side of the flow path 18a communicates with the lumen 13. The prefilled syringe 10 is configured to be capable of discharging a medicinal solution from the tip end surface 18b of the nozzle portion 18.

A syringe barrel 20 including the syringe body 12, the flange portion 16, and the nozzle portion 18 is formed of a transparent resin material or glass, which enables the lumen 13 to be visually recognized. Furthermore, a scale for recognizing the liquid amount of the medicinal solution may be provided at the side portion of the syringe body 12.

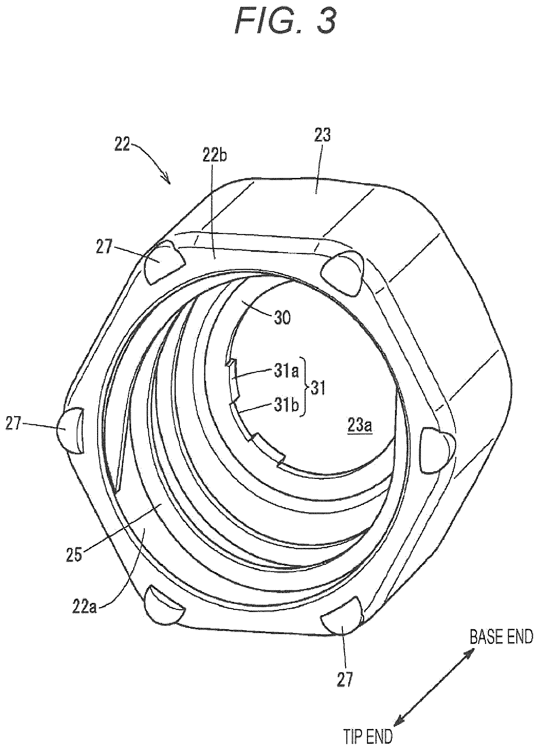

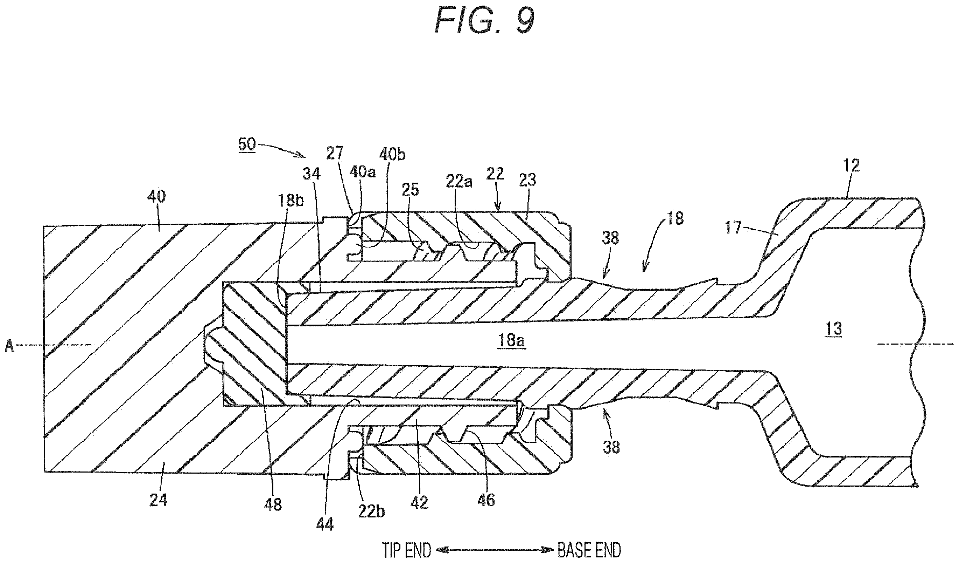

As illustrated in Fig. 1, the lock adapter 22 and a cap 24 are attached to the nozzle portion 18. The lock adapter 22 is a cylindrical member attached to the outer peripheral portion of the nozzle portion 18. As illustrated in Figs. 2 and 3, a female screw 25 is formed on an inner peripheral portion 22a of the lock adapter 22. The cap 24 illustrated in Fig. 1 is detachably screwed into the lock adapter 22. As illustrated in Fig. 9, the cap 24 seals the opening portion of the flow path 18a of the nozzle portion 18 in a state of being screwed into the lock adapter 22. When the cap 24 is detached from the lock adapter 22, the tip end of the nozzle portion 18 is exposed, so that it is enabled to connect a connector of a connection tube, a needle module, or the like to the nozzle portion 18.

As illustrated in Fig. 2, the lock adapter 22 is attached to the outer peripheral portion of the nozzle portion 18. The lock adapter 22 is configured slidable in the axial direction within a range of a slide portion 33 provided in the nozzle portion 18. As illustrated in Fig. 3, the lock adapter 22 includes a cylindrical body portion 23 formed in a substantially hexagonal cylindrical shape. A circular insertion hole 23a is formed inside the cylindrical body portion 23. A female screw 25 is formed on an inner peripheral portion 22a of the cylindrical body portion 23, the inner peripheral portion 22a facing the insertion hole 23a. A tip end surface 22b erected perpendicularly to the axial direction is formed on the tip end side of the cylindrical body portion 23. As illustrated in Fig. 3, the tip end surface 22b is formed in a substantially hexagonal shape when viewed from the tip end side, and six lock protrusions 27 are formed in a portion corresponding to each corner portion of the hexagonal shape so as to protrude toward the tip end. Each lock protrusion 27 has the outer peripheral side formed in a quarter spherical shape. This lock protrusion 27 makes contact with an anti-loosening protrusion 40b (see Fig. 9) of the cap 24 to prevent loosening of the cap 24.

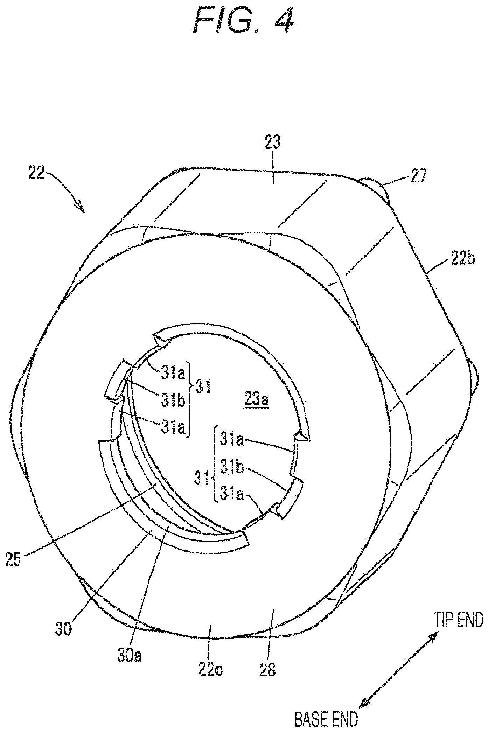

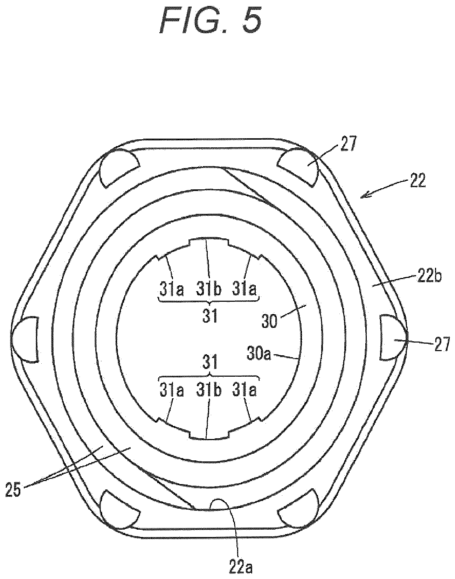

As illustrated in Fig. 4, a base end portion 28 having a base end surface 22c erected perpendicularly to the axial direction is formed on the base end side of the cylindrical body portion 23 of the lock adapter 22. A claw portion 30 is formed to protrude from the base end portion 28 toward the inside of the insertion hole 23a. As illustrated in Fig. 5, the claw portion 30 is formed in a circular ring shape (circumferential shape) when viewed from the axial direction, and is integrally formed over the entire circumferential direction.

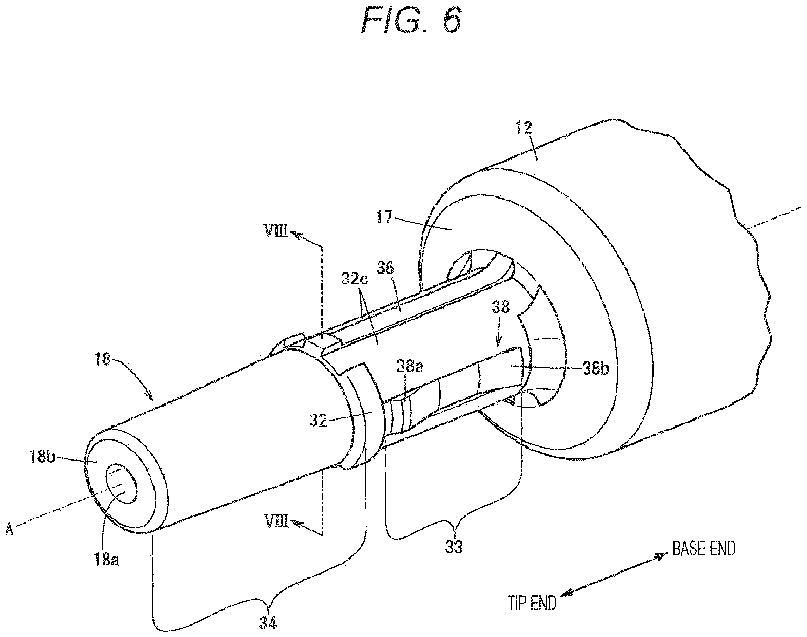

An inner peripheral surface 30a (see Fig. 4) of the claw portion 30 is formed to have an inner diameter slightly larger than the outer diameter of the slide portion 33 in order to make the slide portion 33 slidable. Furthermore, rotation restricting portions 31 are formed at some parts of the claw portion 30 in the circumferential direction. Each of the rotation restricting portions 31 has a pair of rotation restricting protrusions 31a protruding inward from the claw portion 30. The rotation restricting protrusions 31a are formed so as to protrude inward from the inner peripheral surface 30a of the claw portion 30. The pair of rotation restricting protrusions 31a is separated from each other by the same width as that of a rotation restricting rib 36 (see Fig. 6) of the nozzle portion 18 described later, and a slide groove 31b configured slidable over the rotation restricting rib 36 is formed between the pair of rotation restricting protrusions 31a. It should be noted that in order to prevent the claw portion 30 from being divided in the circumferential direction, the position (inner diameter) of the inner periphery of the slide groove 31b is substantially the same as the position (inner diameter) of the inner peripheral surface 30a of the claw portion 30. The rotation restricting protrusions 31a and the slide groove 31b are engaged with the rotation restricting rib 36 to prevent the rotation of the lock adapter 22 in the circumferential direction.

As illustrated in Fig. 2, when the lock adapter 22 is moved to the tip end, the claw portion 30 makes contact with an annular rib 32 to be described later of the nozzle portion 18 to prevent the movement of the lock adapter 22 to the tip end side. That is, the tip end position of the lock adapter 22 is determined by the claw portion 30. When the lock adapter 22 is moved to the tip end position, the lock adapter 22 covers the periphery of a luer tip 34. The cap 24, the needle module, the connector, or the like can be connected to the female screw 25 on the inner peripheral portion 22a. When the lock adapter 22 is moved to the base end side, the most portion of the luer tip 34 is exposed from the lock adapter 22.

In order to ensure compatibility and to prevent erroneous connection, the position of the female screw 25 and the position of the tip end surface 22b of the lock adapter 22 in the tip end position of the lock adapter 22 are defined by a corresponding standard. According to ISO 80369-7, which is a standard related to small-diameter syringes, the axial dimension c from the tip end surface 18b of the nozzle portion 18 to the tip end surface 22b of the lock adapter 22 is defined to fall within 2.100 to 2.573 mm.

As a constituent material of the lock adapter 22, for example, a resin material such as polypropylene, polyethylene, polycarbonate, polyester, or cyclic polyolefin can be used. Among them, a heat-resistant resin such as polypropylene or cyclic polyolefin is preferably used from the viewpoint of preventing deformation during heating in autoclave sterilization.

As illustrated in Fig. 6, the nozzle portion 18 to which the lock adapter 22 is attached is formed on the tip end side of the neck portion 17, which is a reduced diameter portion of the tip end of the syringe body 12. The nozzle portion 18 includes the slide portion 33 formed on the tip end side of the neck portion 17, the luer tip 34 formed on the tip end side of the slide portion 33, and the annular rib 32 provided along a boundary between the slide portion 33 and the luer tip 34.

The slide portion 33 is formed between the tip end of the neck portion 17 and the base end of the annular rib 32. The slide portion 33 is formed in a cylindrical shape having a constant outer diameter size, and has the rotation restricting rib 36 and a slide lock portion 38 on an outer peripheral portion thereof. The rotation restricting rib 36 protrudes outward from the outer peripheral portion of the slide portion 33 and extends in the axial direction. The rotation restricting rib 36 is formed over the entire range in the axial direction of the slide portion 33. The radial protrusion height of the rotation restricting rib 36 is formed lower than the radial protrusion height of the annular rib 32. That is, the rotation restricting rib 36 is formed at such a height that does not divide the annular rib 32 in the circumferential direction. A pair of the rotation restricting ribs 36 is formed spaced apart from each other by 180° in the circumferential direction. It should be noted that the number of the rotation restricting ribs 36 is not limited to two, and may be one or three or more.

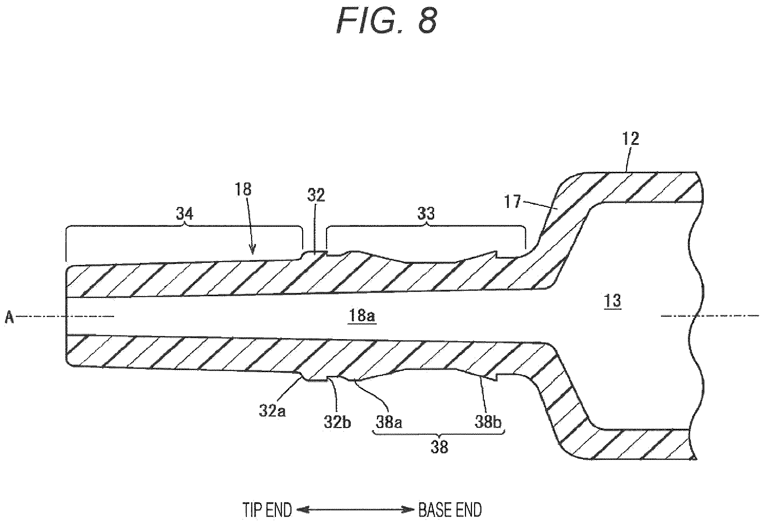

The slide lock portion 38 includes a tip end convex portion 38a for holding the lock adapter 22 at the tip end position and a base end convex portion 38b for holding the lock adapter 22 at the base end position. The tip end convex portion 38a protrudes at substantially the same height as that of the annular rib 32. As illustrated in Fig. 8, the tip end convex portion 38a is configured by an inclined surface in which the tip end side and the base end side are inclined with respect to the axial direction, so that the claw portion 30 of the lock adapter 22 can cross thereover. The tip end convex portion 38a is formed at a position separated from the annular rib 32 by the axial length of the claw portion 30, and makes contact with the base end of the claw portion 30 at the tip end position of the lock adapter 22 to hold the lock adapter 22 at the tip end position. The base end convex portion 38b is formed in the vicinity of the neck portion 17. The base end side of the base end convex portion 38b is configured by a steep surface erected perpendicularly to the axial direction, and engages with the claw portion 30 at the base end position of the lock adapter 22 to hold the lock adapter 22 at the base end position.

As illustrated in Fig. 6, the annular rib 32 is formed on the tip end side of the slide portion 33. The annular rib 32 protrudes to the outer peripheral side of the slide portion 33 and annularly extends in the circumferential direction of the nozzle portion 18. As illustrated in Fig. 8, a tip end surface 32a on the tip end side of the annular rib 32 is configured as an inclined surface inclined in the axial direction in order to facilitate attachment of the lock adapter 22. On the other hand, a base end surface 32b of the annular rib 32 is formed of a surface erecting perpendicularly to the axial direction, and is configured such that the claw portion 30 of the lock adapter 22 cannot cross over the annular rib 32 toward the tip end side.

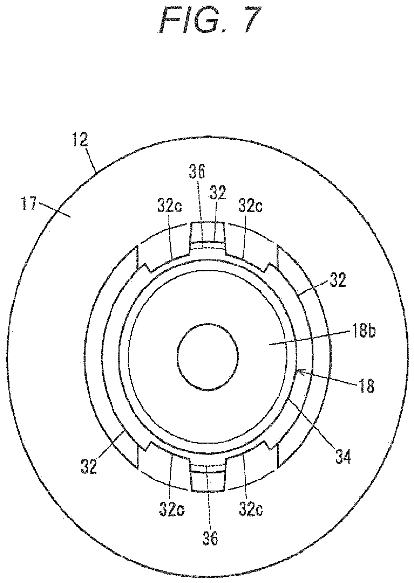

As illustrated in Fig. 6, the protruding height of the annular rib 32 in the outer circumferential direction is higher than that of the rotation restricting rib 36 in the outer circumferential direction. That is, the annular rib 32 has a shape that is divided in the circumferential direction by the rotation restricting rib 36. In addition, as illustrated in Fig. 7, the annular rib 32 has notches 32c each formed by being partially cut out in the circumferential direction in order to facilitate the passage of the rotation restricting protrusion 31a of the lock adapter 22 when the lock adapter 22 is attached. Two notches 32c are provided in each portion adjacent in the circumferential direction to the rotation restricting rib 36.

As illustrated in Fig. 6, the luer tip 34 is formed in a tapered shape the diameter of which gradually decreases toward the tip end. A needle hub of a needle unit and a connector are connected to the luer tip 34.

As illustrated in Fig. 9, the cap 24 is connected to the tip end portion of the nozzle portion 18 and attached so as to cover the luer tip 34. The cap 24 is a member formed in a bottomed cylindrical shape, and includes a body portion 40 having a cylindrical shape provided on the tip end side, an attachment portion 42 extending in a cylindrical shape from the body portion 40 to the base end side, and a luer tip housing portion 44 formed on the inner peripheral side of the attachment portion 42 and the body portion 40. The attachment portion 42 is formed in a cylindrical shape that can be inserted into the lock adapter 22. A male screw 46 that can be screwed into the female screw 25 on the inner peripheral portion 22a of the lock adapter 22 is formed on the outer peripheral portion of the attachment portion 42.

The luer tip housing portion 44 for housing the luer tip 34 is formed on the inner peripheral side of the attachment portion 42. The luer tip housing portion 44 extends to the side of the body portion 40. A seal member 48 is attached to the innermost portion of the luer tip housing portion 44. The seal member 48 is made of a rubber material, and when the cap 24 is screwed into the innermost portion, the seal member is elastically compressed and comes into close contact with the tip end surface 18b of the nozzle portion 18 to seal the flow path 18a.

An opposing surface 40a opposing to the tip end surface 22b of the lock adapter 22 is formed on the base end side of the body portion 40. The opposing surface 40a is provided with an anti-loosening protrusion 40b engaging with the lock protrusion 27 to prevent loosening of the cap 24. When the cap 24 is screwed, the anti-loosening protrusion 40b engages with the lock protrusion 27 to keep the cap 24 in a tightly screwed state. The cap 24 can be formed of a material similar to that of the lock adapter 22.

The syringe assembly 50 of the present embodiment is configured by attaching the lock adapter 22 to the syringe body 12.

The lock adapter 22, the syringe assembly 50, and the prefilled syringe 10 of the present embodiment are configured as described above, and the operation thereof will be described below.

A plurality of syringe assemblies 50 are collectively accommodated in a packaging container called a nested tab container in a sterilized state and distributed, and are provided to a sterile filling facility. In the sterile filling facility, filling of each syringe assembly 50 (syringe body 12) with a medicinal solution and capping with the cap 24 and the gasket 15 are performed. Thereafter, autoclave sterilization is performed on the syringe body 12 in which the medicinal solution is sealed, and the prefilled syringe 10 in Fig. 1 is completed.

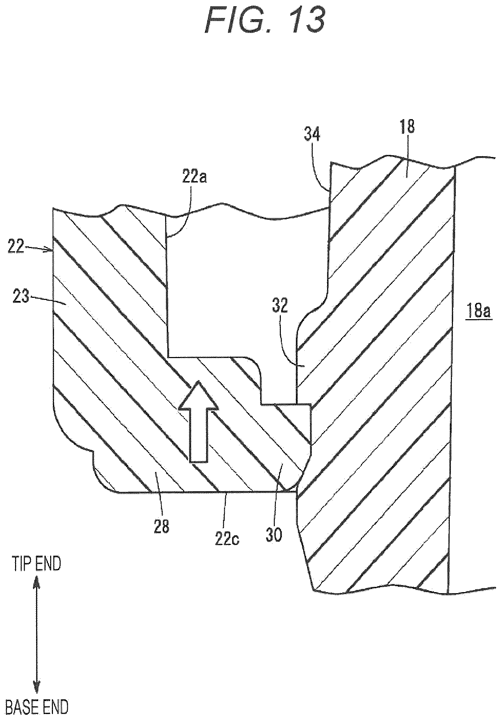

As described above, the autoclave sterilization of the prefilled syringe 10 is performed in a state where the cap 24 is screwed into the lock adapter 22. As illustrated in Fig. 9, in a state where the cap 24 is screwed, a load (tightening load) in the direction in which the cap 24 is separated from the nozzle portion 18 acts by the resilient force of the seal member 48. This tightening load is transmitted to the lock adapter 22 through the male screw 46 of the cap 24. Then, as illustrated in an enlarged view of Fig. 13, the force acts on the claw portion 30 at a base portion of the lock adapter 22 toward a tip end in the axial direction as indicated by a white arrow. Since a high temperature at the time of autoclave sterilization acts on the claw portion 30 under a condition where a tightening load through the cap 24 is acting thereon, the claw portion 30 is easily deformed.

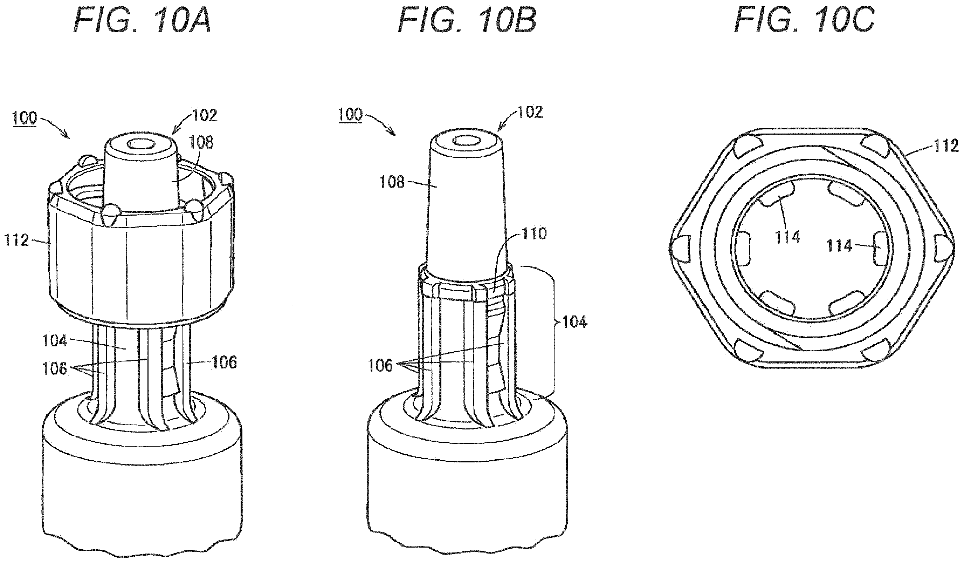

In a prefilled syringe 100 of a comparative example illustrated in Figs. 10A and 10B, six rotation restricting ribs 106 are formed on the outer peripheral portion of a slide portion 104 on the base end side of a nozzle portion 102. The rotation restricting ribs 106 also serve to reinforce a base portion of the nozzle portion 102, and are formed to protrude outward from an annular rib 110 provided along a boundary with a tip portion 108 as illustrated in Fig. 10B. Therefore, the annular rib 110 is divided in the circumferential direction by the rotation restricting ribs 106. As illustrated in Fig. 10C, a lock adapter 112 attached to this nozzle portion 102 has a shape divided in the circumferential direction in such a manner that claw portions 114 on the base end side avoid the rotation restricting ribs 106. Therefore, in the lock adapter 112 of the comparative example, the claw portions 114 have a fragile structure as being divided in the circumferential direction.

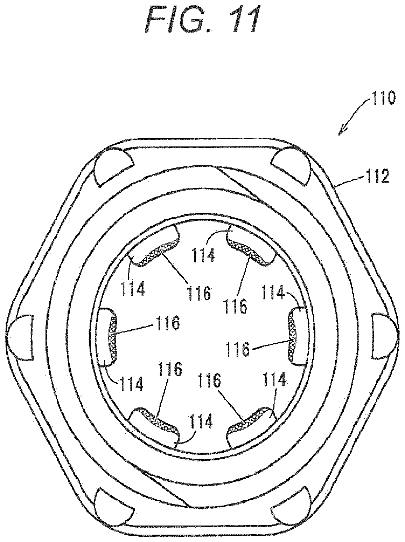

As illustrated in Fig. 11, the claw portions 114 of the lock adapter 112 of the comparative example have a risk of receiving the load of the cap 24 in a state of abutting on the annular rib 110 at contact portions 116 on the inner peripheral side. In this state, the claw portions 114 are liable to be deformed by high temperature due to autoclave sterilization, and a dimensional error may occur.

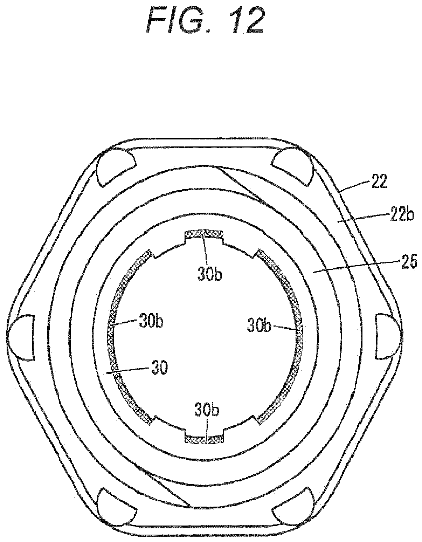

In contrast, in the syringe assembly 50 and the prefilled syringe 10 of the present embodiment, the rotation restricting rib 36 is formed lower than the protruding height of the annular rib 32 in the nozzle portion 18. That is, the rotation restricting rib 36 does not divide the annular rib 32. Therefore, as illustrated in Fig. 12, it is possible to configure the claw portion 30 of the lock adapter 22 to have a shape integrally connected in the circumferential direction, so that the rigidity of the claw portion 30 can be enhanced. As illustrated, in the lock adapter 22 of the present embodiment, the contact portion 30b makes contact with the annular rib 32. In this manner, the tightening load of the cap 24 is dispersed over a wide range of the claw portion 30.

As a result, as illustrated in Fig. 13, even when autoclave sterilization is performed in a state where the tightening load of the cap 24 acts on the claw portion 30, deformation of the claw portion 30 can be suppressed. Furthermore, variations in the axial position of the tip end surface 22b of the lock adapter 22 and the axial position of the female screw 25 are suppressed.

The lock adapter 22, the syringe assembly 50, and the prefilled syringe 10 of the present embodiment have the following effects.

The lock adapter 22 of the present embodiment relates to the lock adapter 22 attached on the periphery of the nozzle portion 18 at the tip end of the prefilled syringe 10 and configured slidable in the axial direction of the nozzle portion 18. The lock adapter 22 includes: the cylindrical body portion 23 formed in a cylindrical shape surrounding the periphery of the nozzle portion 18; and the claw portion 30 protruding to the inner peripheral side from the base end portion 28 of the cylindrical body portion 23, and configured to determine the tip end position of the lock adapter 22 by being caught in the annular rib 32 extending in the circumferential direction of the nozzle portion 18. The claw portion 30 is formed in a circumferential shape when viewed from the axial direction and is integrally connected in the circumferential direction.

With the above configuration, the rigidity of the claw portion 30 can be enhanced. In addition, the tightening load of the cap 24 is dispersed over a wide range in the circumferential direction of the claw portion 30. As a result, even when autoclave sterilization is performed in a state where the tightening load of the cap 24 acts on the claw portion 30, deformation of the claw portion 30 can be prevented, and variations in the axial position of the tip end surface 22b of the lock adapter 22 and the axial position of the female screw 25 can be suppressed.

The lock adapter 22 may further include the rotation restricting protrusion 31a that protrudes inward from the claw portion 30 and engages with the rotation restricting rib 36 extending in the axial direction of the nozzle portion 18. With this configuration, it is possible to realize a configuration capable of preventing the rotation of the lock adapter 22 without dividing the claw portion 30 in the circumferential direction.

The syringe assembly 50 of the present embodiment includes the syringe body 12 having the lumen 13 capable of accommodating a medicinal solution, the nozzle portion 18 formed at the tip end of the syringe body 12, and the lock adapter 22 attached to the outer periphery of the nozzle portion 18. In the syringe assembly 50, the nozzle portion 18 includes the slide portion 33 provided on the base end side and on which the lock adapter 22 is slidable in the axial direction, the luer tip 34 provided on the tip end side of the slide portion 33, and the annular rib 32 extending and protruding in the circumferential direction along the boundary between the luer tip 34 and the slide portion 33. The lock adapter 22 includes the cylindrical body portion 23 formed in a cylindrical shape surrounding the periphery of the nozzle portion 18, and the claw portion 30 protruding to the inner peripheral side from the base end portion 28 of the cylindrical body portion 23, and configured to determine a tip end position of the lock adapter 22 by being caught in the annular rib 32 extending in the circumferential direction of the nozzle portion 18. The claw portion 30 of the lock adapter 22 is formed in a circumferential shape when viewed from the axial direction and is integrally connected in the circumferential direction.

With the above-described syringe assembly 50, the deformation of the claw portion 30 can be prevented, and variations in the axial position of the tip end surface 22b of the lock adapter 22 and the axial position of the female screw 25 can be suppressed.

In the syringe assembly 50 described above, the rotation restricting rib 36 extending in the axial direction is formed on the slide portion 33 of the nozzle portion 18, and the protruding height of the rotation restricting rib 36 may be formed lower than the protruding height of the annular rib 32. With the above-described configuration, since it is not necessary to form the claw portion 30 in the circumferential shape of the lock adapter 22 so as to avoid the rotation restricting rib 36, the claw portion 30 can be integrally formed. As a result, the strength of the claw portion 30 is improved, and deformation of the claw portion 30 can be suppressed.

In the syringe assembly 50 described above, the rotation restricting rib 36 may be formed inward of the claw portion 30 of the lock adapter 22. With this configuration, since the rotation restricting rib 36 is positioned inside the claw portion 30, the rotation restricting rib 36 does not interfere with the claw portion 30, and the claw portion 30 can be integrally formed in the circumferential direction.

In the syringe assembly 50 described above, the rotation restricting protrusion 31a may be formed that protrudes inward from the inner peripheral surface 30a of the claw portion 30 of the lock adapter 22 and engages with the rotation restricting rib 36. With this configuration, rotation of the lock adapter 22 can be prevented.

In the syringe assembly 50 described above, the notch 32c for allowing the rotation restricting protrusion 31a to pass therethrough may be formed at a portion adjacent in the circumferential direction to the rotation restricting rib 36 of the annular rib 32. With this configuration, upon attaching the lock adapter 22 to the nozzle portion 18, the rotation restricting protrusion 31a can easily pass through the annular rib 32, which makes the assembly easy. Furthermore, it is possible to prevent breakage of the annular rib 32 and the rotation restricting protrusion 31a at the time of assembly.

In the syringe assembly 50 described above, the tip end surface 32a of the annular rib 32 is gradually reduced in diameter and inclined toward the tip end side, and the base end surface 32b of the annular rib 32 may be erected perpendicularly to the axial direction. With this configuration, upon attaching the lock adapter 22 to the nozzle portion 18, the claw portion 30 can easily cross over the annular rib 32, which is preferable. In addition, since the base end surface 32b of the annular rib 32 is formed to be erected upright, even when the tightening load of the cap 24 or the like acts on the claw portion 30, it is possible to prevent the claw portion 30 from falling off from the annular rib 32 to the tip end side, which is preferable.

The prefilled syringe 10 of the present embodiment includes the syringe body 12 accommodating a medicinal solution in the lumen 13, the gasket 15 to be inserted from the base end side of the syringe body 12 to seal the lumen 13, the nozzle portion 18 formed at the tip end of the syringe body 12, the lock adapter 22 attached to the outer periphery of the nozzle portion 18, and the cap 24 screwed into the lock adapter 22 to seal the nozzle portion 18. The nozzle portion 18 of this prefilled syringe 10 includes the slide portion 33 provided on the base end side and on which the lock adapter 22 is slidable in the axial direction, the luer tip 34 provided on the tip end side of the slide portion 33, and the annular rib 32 extending and protruding in the circumferential direction along the boundary between the luer tip 34 and the slide portion 33. The lock adapter 22 includes the cylindrical body portion 23 formed in a cylindrical shape surrounding the periphery of the nozzle portion 18, and the claw portion 30 protruding to the inner peripheral side from the base end portion 28 of the cylindrical body portion 23, and configured to determine the tip end position of the lock adapter 22 by being caught in the annular rib 32 extending in the circumferential direction of the nozzle portion 18. The claw portion 30 of the lock adapter 22 is formed in a circumferential shape when viewed from the axial direction and is integrally connected in the circumferential direction.

With the prefilled syringe 10 configured as described above, the deformation of the claw portion 30 can be prevented, and variations in the axial position of the tip end surface 22b of the lock adapter 22 and the axial position of the female screw 25 can be suppressed.

Although the present invention has been described above with reference to preferred embodiments, the present invention is not limited to the above embodiments, and it goes without saying that various modifications can be made without departing from the gist of the present invention. For example, in the present embodiment, the lock adapter may be separate from the prefilled syringe and configured to slide, or may be separate and configured not to slide.

## Claims

1. A lock adapter attached to a periphery of a nozzle portion at a tip end of a prefilled syringe and configured slidable in an axial direction of the nozzle portion, the lock adapter comprising:
a cylindrical body portion formed in a cylindrical shape surrounding the periphery of the nozzle portion; and
a claw portion protruding to an inner peripheral side from a base end portion of the cylindrical body portion, and configured to determine a tip end position of the lock adapter by being caught in an annular rib extending in a circumferential direction of the nozzle portion, wherein
the claw portion is formed in a circumferential shape when viewed from the axial direction and is integrally connected in the circumferential direction.

2. The lock adapter according to claim 1, further comprising a rotation restricting protrusion that protrudes inward from the claw portion and engages with a rotation restricting rib extending in the axial direction of the nozzle portion.

3. A syringe assembly comprising:
a syringe body having a lumen capable of accommodating a medicinal solution;
a nozzle portion formed at a tip end of the syringe body; and
a lock adapter attached to an outer periphery of the nozzle portion, wherein
the nozzle portion includes: a slide portion provided on a base end side and on which the lock adapter is slidable in an axial direction; a luer tip provided on a tip end side of the slide portion; and an annular rib extending and protruding in a circumferential direction along a boundary between the luer tip and the slide portion,
the lock adapter includes: a cylindrical body portion formed in a cylindrical shape surrounding the periphery of the nozzle portion; and a claw portion protruding to an inner peripheral side from a base end portion of the cylindrical body portion, and configured to determine a tip end position of the lock adapter by being caught in the annular rib extending in the circumferential direction of the nozzle portion, and
the claw portion of the lock adapter is formed in a circumferential shape when viewed from the axial direction and is integrally connected in the circumferential direction.

4. The syringe assembly according to claim 3, wherein the slide portion of the nozzle portion is provided with a rotation restricting rib extending in the axial direction, and a protruding height of the rotation restricting rib is formed lower than a protruding height of the annular rib.

5. The syringe assembly according to claim 4, wherein the rotation restricting rib is formed inward of the claw portion of the lock adapter.

6. The syringe assembly according to claim 4 or 5, wherein a rotation restricting protrusion protruding inward from an inner peripheral portion of the claw portion of the lock adapter so as to engage with the rotation restricting rib is formed.

7. The syringe assembly according to claim 6, wherein a notch that allows the rotation restricting protrusion to pass therethrough is formed at a portion of the annular rib adjacent in the circumferential direction to the rotation restricting rib.

8. The syringe assembly according to claim 3, wherein a tip end surface of the annular rib is gradually reduced in diameter and inclined toward the tip end side, and a base end surface of the annular rib is erected perpendicularly to the axial direction.

9. A prefilled syringe comprising:
a syringe body having a lumen accommodating a medicinal solution;
a gasket to be inserted from a base end side of the syringe body to seal the lumen;
a nozzle portion formed at a tip end of the syringe body;
a lock adapter attached to an outer periphery of the nozzle portion; and
a cap screwed into the lock adapter to seal the nozzle portion, wherein
the nozzle portion includes: a slide portion provided on a base end side and on which the lock adapter is slidable in an axial direction; a luer tip provided on a tip end side of the slide portion; and an annular rib extending and protruding in a circumferential direction along a boundary between the luer tip and the slide portion;
the lock adapter includes: a cylindrical body portion formed in a cylindrical shape surrounding a periphery of the nozzle portion; and a claw portion protruding to an inner peripheral side from a base end portion of the cylindrical body portion, and configured to determine a tip end position of the lock adapter by being caught in the annular rib extending in the circumferential direction of the nozzle portion, and
the claw portion of the lock adapter is formed in a circumferential shape when viewed from the axial direction and is integrally connected in the circumferential direction.
